# EUROPEAN PATENT SPECIFICATION

(11) **EP 3 541 059 B1**
(45) Date of publication and mention of the grant of the patent: **04.01.2023**
(21) Application number: 16921435.0
(22) Date of filing: 14.11.2016
(51) Int. Cl.: H04N 1/40, B41J 3/407, H04N 1/409, H04N 1/00, H04N 1/60, B41J 29/38, A45D 34/04, A45D 44/00, A61M 37/00, B41J 3/36

(54) **MOBILE IMAGE-FORMING DEVICE, IMAGE CORRECTING METHOD THEREOF, AND NON-TRANSITORY COMPUTER-READABLE RECODING MEDIUM**
MOBILE BILDERZEUGUNGSVORRICHTUNG, BILDKORREKTURVERFAHREN DAFÜR UND ÜBERGANGSLOSES COMPUTERLESBARES AUFZEICHNUNGSMEDIUM
DISPOSITIF DE FORMATION D'IMAGE MOBILE, PROCÉDÉ DE CORRECTION D'IMAGE ASSOCIÉ, ET SUPPORT D'ENREGISTREMENT LISIBLE PAR ORDINATEUR NON TRANSITOIRE

(43) Date of publication of application: 18.09.2019
(73) Proprietor: Prinker Korea Inc., Gyeonngi-do 16426 (KR)
(72) Inventor: LEE, Jong In, Seoul 07621 (KR); YUN, Tae Sik, Seoul 06767 (KR); LEE, Kyu Suk, Suwon-si Gyeonggi-do 16687 (KR)
(74) Representative: Manitz Finsterwald Patent- und Rechtsanwaltspartnerschaft mbB
(86) International application number: PCT/KR2016/013055
(87) International publication number: WO 2018/088594

(56) References cited:
- KR-A- 20170 009 261
- US-A1- 2004 078 278
- US-A1- 2012 067 364
- US-A1- 2013 066 170
- US-A1- 2014 300 676
- US-B1- 6 341 831
- US-B1- 8 083 422
- US-B1- 8 322 816

## Description

### TECHNICAL FIELD

The present disclosure relates to a mobile image-forming device and an image correcting method. The mobile image-forming device is configured to correct an image so that the image can be printed on skin to be similar to an original image by reflecting the condition and curvature of skin surface on which the image is to be printed.

### BACKGROUND

In recent years, as people become increasingly interested in tattoos, body painting, and the like, there is an increasing demand for printers capable of printing on skin. Tattoos or body painting must be performed by experts and thus lack accessibility. Using a mobile printer provides an advantage that a user can easily print a desired image on skin and an advantage that various images can be used.

However, since a mobile printer prints an image on skin, there has been a great difference between an original image to be printed and a result image printed on the skin. Specifically, depending on the skin color and condition of the skin used as the background of the image, there may be a difference in color tone between the original image and the result image. Further, the shape of the printed image may be distorted due to curves of the human body, unlike when printing an image on a plane surface.

In this regard, a device and method for automatically treating makeup on the face have previously been proposed and examples are disclosed in US 2004/0078278 A1 (Title: Cosmetic treatment method and device, in particular for care, make-up or colouring) and US 2012/0067364 A1 (Title: Facial make-up application machine and make-up application method using the same). These documents do not disclose a configuration that measures the moisture of the skin and adjusts the printing state based on it. A device and method for measuring the moisture of skin is disclosed in US 2013/066170 A1 (Title: Sensing Moisture Level of Human Skin).

### DISCLOSURE OF THE INVENTION

### PROBLEMS TO BE SOLVED BY THE INVENTION

To solve the above-described problem, the present disclosure provides a mobile image-forming device according to independent claim 1 and an image correcting method according to independent claim 8.

### MEANS FOR SOLVING THE PROBLEMS

According to an embodiment of the present disclosure, a mobile image-forming device includes a surface measuring unit configured to measure a contact area with the mobile image-forming device in a surface area on which a print image is to be printed, an image processing unit configured to extract moisture data from the measured result and correct the print image based on the extracted moisture data, and an image forming unit configured to print the corrected print image on the surface.

Further, the surface measuring unit may include a location sensor configured to determine the contact area with the mobile image-forming device and an image sensor configured to photograph an image of a contact area with the location sensor, and the image processing unit may analyze the photographed image to extract at least one of contrast data and curvature data and correct the print image based on at least one of the extracted contrast data and curvature data.

Furthermore, the surface measuring unit may further include a moisture sensor configured to measure a moisture content or hydrogen ion concentration (pH) of the contact area, and the image processing unit may correct an ink discharge amount for the print image based on the measured moisture content or hydrogen ion concentration.

Moreover, the location sensor may be arranged on a horizontal and vertical line of where the image forming unit is placed, and a two-dimensional motion of the mobile image forming device may be detected using the arranged location sensor.

Further, the image processing unit may correct a color of the print image using a pre-stored conversion table based on the extracted contrast data.

Furthermore, the image processing unit may correct a discharge amount of white (W) ink among cyan (C), magenta (M), yellow (Y), and W inks to increase as the brightness of the extracted contrast data decreases.

Moreover, the image processing unit may analyze the photographed image to detect the curvature of the contact area and perform a three-dimensional rendering on the two-dimensional print image according to the detected curvature.

Further, the image forming unit may include a head configured to discharge ink, and a coating unit configured to spray a coating agent before and after the ink is discharged from the head.

Furthermore, the mobile image-forming device my further include a display unit and a user interface configured to receive a user input, and the image processing unit may control the display unit to compose the print image on the surface area on which the print image is to be printed and to display the composed image, and when a user input for adjusting a location or size of the displayed image is received, the image processing unit may correct the print image in response to the received user input.

Moreover, the mobile image-forming device my further include a camera configured to photograph an image, and the photographed image may be used as the print image.

Further, the mobile image-forming device my further include a communication interface, and the image processing unit may control the communication interface to remove a skin color from the photographed image and to search online for an image that is similar to the image from which the skin color has been removed, and the searched image may be used as the print image.

According to another embodiment of the present disclosure, an image correcting method of a mobile image-forming device includes measuring a contact area with the mobile image-forming device in a surface area on which a print image is to be printed, extracting moisture data from the measured result, correcting the print image based on the extracted moisture data, and printing the corrected print image on the surface.

Further, the extracting may include photographing an image of the contact area and analyzing the photographed image to extract at least one of contrast data and curvature data, and the correcting may include correcting the print image based on at least one of the extracted contrast data and curvature data.

Furthermore, the measuring may further include measuring a moisture content or hydrogen ion concentration (pH) of the contact area, and the correcting may include correcting an ink discharge amount for the print image based on the measured moisture content or hydrogen ion concentration.

Moreover, the correcting may include correcting a color of the print image using a pre-stored conversion table based on the extracted contrast data.

Further, the correcting may include correcting a discharge amount of white (W) ink among cyan (C), magenta (M), yellow (Y), and W inks, to increase as the brightness of the extracted contrast data decreases.

Furthermore, the correcting may include analyzing the photographed image to detect the curvature of the contact area and performing a three-dimensional rendering on the two-dimensional print image according to the detected curvature.

Moreover, the correcting may further include composing the print image on the surface area on which the print image is to be printed and displaying the composed image, and when a user input for adjusting a location or size of the displayed image is received, correcting the print image in response to the received user input.

Further, the image correcting method may further include photographing an image, removing a skin color from the photographed image, and searching online for an image that is similar to the image from which the skin color has been removed, and the searched image may be used as the print image.

According to yet another embodiment of the present disclosure, a non-transitory computer-readable recording medium including a program for performing an image correcting method of a mobile image-forming device, and the image correcting method includes measuring a contact area with the mobile image-forming device in a surface area on which a print image is to be printed, extracting at least one of curvature data and moisture data from the measured result, correcting the print image based on at least one of the extracted curvature data and moisture data, and printing the corrected print image on the surface.

### EFFECTS OF THE INVENTION

According to various embodiments of the present disclosure as described above, it is possible to correct a print image being printed on skin to be similar to an original image as much as possible by reflecting the skin color, body curves and skin conditions that differ from person to person.

### BRIEF DESCRIPTION OF THE DRAWINGS

**FIG. 1** is a schematic block diagram provided to explain the configuration of a mobile image-forming device according to an embodiment of the present disclosure,
**FIG. 2** is a block diagram provided to explain in detail the configuration of the mobile image-forming device according to an embodiment of the present disclosure,
**FIG. 3** is a conceptual diagram showing an operation of the mobile image-forming device according to an embodiment of the present disclosure,
**FIG. 4** illustrates that moisture data are displayed on a display unit according to an embodiment of the present disclosure,
**FIG. 5** is provided to explain the arrangement of a location sensor according to an embodiment of the present disclosure,
**FIG. 6a** through **FIG. 6C** are provided to explain a coating unit according to an embodiment of the present disclosure,
**FIG. 7** is provided to explain an air blower and a roller sensor according to an embodiment of the present disclosure,
**FIG. 8** is provided to explain a roller sensor according to an embodiment of the present disclosure,
**FIG. 9** and **FIG. 10** are provided to explain a cleaning unit according to an embodiment of the present disclosure,
**FIG. 11** is provided to explain photographing an image and determining a print image through searching according to an embodiment of the present disclosure,
**FIG. 12** is provided to explain a member configured to maintain a constant distance between skin and an image forming unit according to an embodiment of the present disclosure,
**FIG. 13** is a conceptual diagram provided to explain the mobile image-forming device which operates and interworks with a mobile device according to another embodiment of the present disclosure, and
**FIG. 14** and **FIG. 15** are flowcharts provided to explain an image correcting method of a mobile image-forming device according to various embodiments of the present disclosure.

### BEST MODE FOR CARRYING OUT THE INVENTION

Hereafter, example embodiments will be described in detail with reference to the accompanying drawings. However, it is to be noted that the present disclosure is not limited to the example embodiments but can be embodied in various other ways. In the drawings, parts irrelevant to the description are omitted for the simplicity of explanation, and like reference numerals denote like parts through the whole document Further, if it is considered that description of related known configuration or function may cloud the gist of the present disclosure, the description thereof will be omitted. Further, the terms used herein are defined in consideration of functions in the present disclosure, and may vary depending on intentions of a user or operator, or usual practices. Therefore, the definitions thereof should be construed based on the contents throughout the specification.

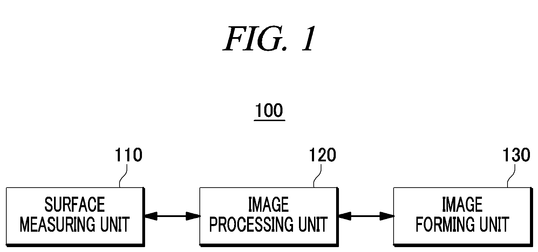

**FIG. 1** is a block diagram illustrating the configuration of a mobile image-forming device 100 according to an embodiment of the present disclosure. Referring to **FIG. 1****,** the mobile image-forming device 100 includes a surface measuring unit 110, an image processing unit 120, and an image forming unit 130.

Herein, the mobile image-forming device 100 may be configured such that the mobile image-forming device 100 may be held and moved by a user as the mobile image-forming device 100 performs a printing function on a surface on which the user intends to print an image. Unlike a general image-forming device that is configured to print a two-dimensional image as a print medium (for example, paper) and an ink cartridge each makes a one-dimensional motion, the mobile image-forming device 100 according to an embodiment of the present disclosure is configured such that a print medium (for example, skin of a human body) does not move and a head 131 of the mobile image-forming device 100 makes a two-dimensional motion while the user moves the mobile image-forming device 100 so as to print a two-dimensional image. Further, the mobile image-forming device 100 may use an ink composition of cyan (C), magenta (M), yellow (Y), and white (W) instead of C, M, Y, and black (K) due to the fact that the mobile image-forming device 100 prints an image on skin. However, there is no limitation that the mobile image-forming device 100 has to use an ink composition of C, M, Y, and W only.

The mobile image-forming device 100 is portable, and is characterized in that the print medium is not limited to a certain size of paper. Therefore, an image can be easily printed on a variety of places, such as human skin, fabric, and the like. Hereafter, an example of printing an image on human skin will be mainly described, but the print medium is not necessarily limited to human skin.

The surface measuring unit 110 may measure the state of a surface area on which a print image is to be printed. For example, the surface measuring unit 110 may measure a contact area with the mobile image-forming device 100 in the surface area. The surface measuring unit 110 include various measuring devices, such as a location sensor, an image sensor, a moisture sensor, an optical sensor, and the like.

The surface measuring unit 110 may determine a contact area with the mobile image-forming device 100, photograph an image of the contact area, and measure a moisture content or hydrogen ion concentration (pH) of the contact area.

The image processing unit 120 may correct a print image according to the condition of a surface on which the print image is to be printed. For example, the image processing unit 120 may extract at least one of contrast data, curvature data, and moisture data based on the results measured by the surface measuring unit 110. In addition, the image processing unit 120 may correct the print image based on the extracted data.

For example, the image processing unit 120 may correct a color and tone of the print image based on the contrast data. The image processing unit 120 may correct the color of the print image using a pre-stored conversion table. If the skin of the area on which an image is to be printed is dark, the image processing unit 120 may increase a discharge amount of W ink.

In another example, the image processing unit 120 may perform a three-dimensional rendering on a two-dimensional print image according to the curvature of where the print image is to be printed based on the curvature data. In the case of printing an image on the arm, the image processing unit 120 may distort the shape of a plane print image to look as if the plane print image is wound around the arm.

In yet another example, the image processing unit 120 may correct an ink discharge amount for the print image based on moisture data. The moisture data may include a moisture content, a hydrogen ion concentration (pH), and the like. The image processing unit 120 may correct the print image by increasing the ink discharge amount in case of dry skin and decreasing the ink discharge amount in case of oily skin.

The image forming unit 130 may print a print image on a surface. A general image-forming device prints an image by discharging ink for each one-dimensional line, whereas the image forming unit 130 of the mobile image-forming device 100 according to an embodiment of the present disclosure prints an image while making a two-dimensional motion.

The image forming unit 130 may further include a head 131 for discharging ink and a coating unit 133 configured to spray a coating agent before and after the discharge of ink in order to improve the print quality. The reason for spraying a coating agent is that an image printed on skin or fabric, and the like may be degraded in print quality in terms of ink adhesion, color reproducibility, quick dryness, and wear resistance, compared to printing an image on paper.

With the above-described mobile image-forming device 100, it is possible to correct a print image being printed on skin to be similar to an original image as much as possible by reflecting the skin color, body curves and skin conditions that differ from person to person.

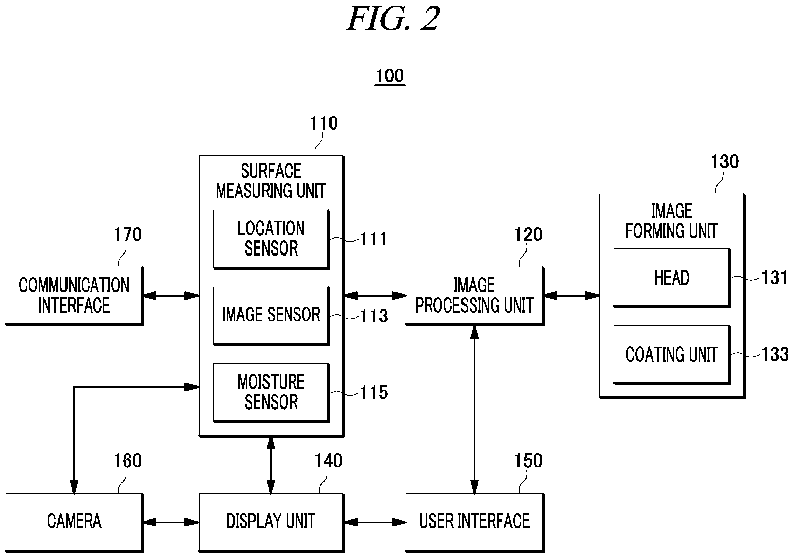

**FIG. 2** is a block diagram provided to explain in detail the configuration of the mobile image-forming device 100 according to an embodiment of the present disclosure. Referring to **FIG. 2****,** the mobile image-forming device 100 may include the surface measuring unit 110, the image processing unit 120, the image forming unit 130, a display unit 140, a user interface 150, a camera 160, and a communication interface 170.

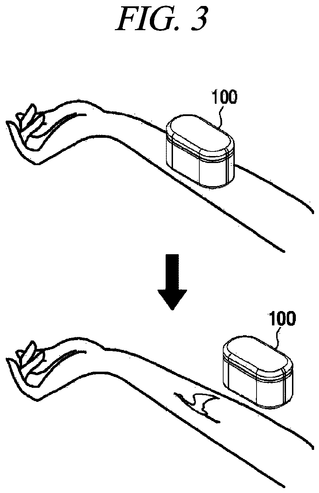

The surface measuring unit 110 may measure a contact are with the mobile image-forming device 100 in a surface area on which a print image is to be printed. As shown in **FIG. 3****,** the mobile image-forming device 100 may print a desired image on the skin while being in contact with a part of the user's body. Therefore, there exists a need to correct the print image by analyzing the contact area with the mobile image-forming device 100. The surface measuring unit 110 may be implemented with various measuring devices. For example, the surface measuring unit 110 may include a location sensor 111, an image sensor 113, and a moisture sensor 115.

The location sensor 111 may perform a function of identifying a location of the mobile image-forming device 100. The location sensor 111 may determine the contact area with the mobile image-forming device 100. Furthermore, the location sensor 111 may detect a location where the mobile image-forming device 100 is moved on the surface.

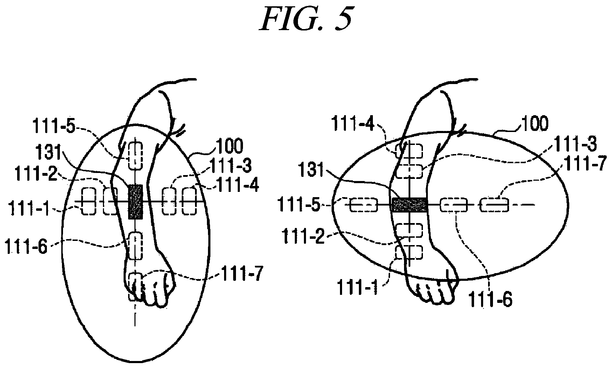

Since the mobile image-forming device 100 makes a two-dimensional motion, at least one location sensor is needed in each of vertical and horizontal directions with respect to the image forming unit 130. For example, motion of the mobile image-forming device 100 may be detected using a first location sensor 111 configured to detect an up/down motion and a second location sensor 111' configured to detect a left/right motion. The arrangement of the location sensor 111 will be described again with reference to **FIG. 5****.**

The image sensor 113 may perform a function of photographing an image of a contact area with the mobile image-forming device 100. Based on the image photographed in the image sensor 113, the image processing unit 120 may analyze the contrast or curvature of the surface based on the image photographed by the image sensor 113.

For example, the image sensor 113 may be implemented as a charge coupled device (CCD) element or complementary metal oxide semiconductor (CMOS) element. A CCD is an element where each metal-oxide-silicon (MOS) capacitor is arranged very closely and where an electron carrier is stored in a capacitor and is transferred. A CMOS image sensor is an element configured to adopt a switching method in which MOS transistors are created as many as pixels by using the CMOS technology using a control circuit and a signal processing circuit as peripheral circuits and used to sequentially detect the output.

If the location sensor 111 is implemented as a sensor using an optical method to detect the contrast or curvature of a print surface, the location sensor 111 may be implemented to substitute for the image sensor 113.

The moisture sensor 115 may measure a moisture content of a print surface. For example, the moisture sensor 115 may measure a moisture content of the stratum corneum of the skin. Furthermore, the moisture sensor 115 may measure a hydrogen ion concentration (pH) of the skin. The image processing unit 120 may adjust the ink discharge amount for a print image using the moisture data measured by the moisture sensor 115. The criteria for determining skin types will be described below.

The moisture sensor 115 may measure the moisture content or hydrogen ion concentration based on changes in capacitance or resistance value. For example, the moisture sensor 115 may be implemented to include two metallic measuring units. For the sake of easy measurement, the moisture sensor 115 may be implemented to include a measuring unit that protrudes from the moisture sensor 115. In this case, the moisture sensor 115 may be configured to protrude only when the user measures moisture data. That is, in an embodiment of the present disclosure, the mobile forming apparatus 100 may be configured to measure only contrast data or curvature data and the moisture sensor 115 may operate only when detailed skin information is needed.

The image processing unit 120 may extract at least one of contrast data, curvature data and moisture data based on the results measured by the surface measuring unit 110. Furthermore, the image processing unit 120 may correct the print image based on the extracted data.

The image processing unit 120 may analyze a skin image photographed by the surface measuring unit 110 and extract contrast data. For example, the image processing unit 120 may correct the color, tone or contrast of the print image based on the extracted contrast data. If the skin on which an image is to be printed is dark (low brightness), the ink discharge amount needs to be increased in order to print the image distinctly. Therefore, the image processing unit 120 may adjust the ink discharge amount to increase as the brightness of the extracted contrast data decreases. The image processing unit 120 may be implemented to increase only the discharge amount of W ink among C, M, Y, and W inks in order to increase the brightness.

The image processing unit 120 may use a pre-stored conversion table in order to adjust the color, tone, and contrast of the print image. The image processing unit 120 may extract data for differentiating light and darkness and differentiate color tones of the skin by comparing the extracted data with a reference value stored in the conversion table. Furthermore, the image processing unit 120 may correct a color tone of the print image using the pre-stored conversion table. If the extracted contrast data are the same as a default value, the image processing unit 120 may skip correcting the contrast.

The image processing unit 120 may analyze the photographed image and extract curvature data. Furthermore, the image processing unit 120 may correct the shape of the print image using the extracted curvature data. The image processing unit 120 may analyze the photographed image to detect the curvature of where the image is to be printed, and perform a three-dimensional rendering on the two-dimensional print image according to the detected curvature of the area. For example, the image processing unit 120 may analyze a distance of a print position on the photographed image and identify the extent of the curvature.

In another example, the image processing unit 120 may analyze the photographed image to determine which part of the body corresponds to the photographed image, and predict the curvature of the print position and correct the print image. In the case as show in **FIG. 3****,** an image is to be printed on the arm, and, thus, the image processing unit 120 may predict the curvature of the arm and correct the print image. If user information is previously input, the image processing unit 120 may combine information, such as the gender, height, weight, and the like with information on the body part and predict the extent of the curvature.

If a plane print image is printed as it is without considering the curvature, an image different from what is intended by the user may be printed. Therefore, even when a plane image is printed on a curved skin, the image processing unit 120 corrects the print image using the curvature data in order to minimize deformation of the original image. In the case of printing an image on the arm as illustrated in **FIG. 3****,** the image processing unit 120 may distort the shape of a plane print image to look as if the plane print image is wound around the arm.

The image processing unit 120 may correct the ink discharge amount for the print image based on the measured moisture content or hydrogen ion concentration. For example, the image processing unit 120 may determine a skin type according to measured values as shown in Table 1 below and correct the ink discharge amount according to the determined skin type.

**[Table 1]**

| Measured value | Skin type | Ink discharge amount |
|---|---|---|
| Moisture content: less than 15% pH: more than 6.5 | Dry | Twice the default value |
| Moisture content: 15% to 30% pH: 5 to 6.5 | Normal | Default value |
| Moisture content: more than 30% pH: less than 5 | Oily | Half the default value |

The determination criteria shown in Table 1 are jus an example, and skin types may be determined according to other determination criteria.

Human skin needs enzymes, such as phospholipase and β-gluco-cerebrosidase. The hydrogen ion concentration of skin differs depending on the ratio of these enzymes. As the hydrogen ion concentration decreases, the skin becomes oily.

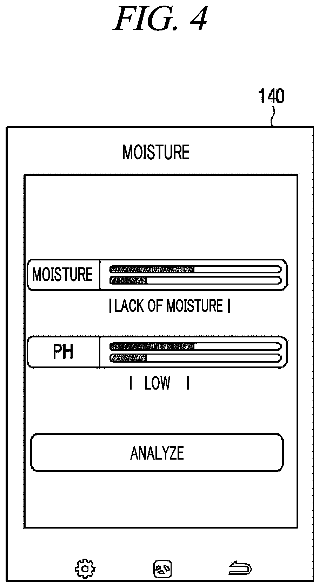

**FIG. 4** illustrates moisture data analysis result displayed on the display unit 140. The moisture content and hydrogen ion concentration may be displayed while being changed in real time according to the measurement result. Furthermore, in response to a user command for pressing an analyze button, the image processing unit 120 may determine a skin type according to a predetermined condition.

If the determined skin type is dry, the image processing unit 120 may adjust the ink discharge amount to increase. If the determined skin type is oily, the image processing unit 120 may adjust the ink discharge amount to decrease.

The image forming unit 130 may print a print image on the surface of skin. The image forming unit 130 may include the head 131 for discharging ink and the coating unit 133 configured to spray a coating agent.

The head 131 discharges ink according to the determined type and discharge amount of ink based on print data. For example, ink to be used on the skin may include four types: C, M, Y, and W.

**FIG. 5** illustrates the arrangement of multiple location sensors 111-1, 111-2, 111-3, 111-4, 111-5, 111-6, and 111-7 of the head 131. Referring to **FIG. 5****,** the multiple location sensors 111-1, 111-2, 111-3, 111-4, 111-5, 111-6, and 111-7 may be arranged on the image forming unit 130, more specifically on horizontal and vertical lines of where the head 131 is arranged. Through the location sensors 111 arranged in horizontal and vertical directions, two-dimensional motions of the mobile image-forming device 100 may be detected.

In order to print a print image on the skin surface with the mobile image-forming device 100, the mobile image-forming device 100 needs to be moved such that the head 131 moves over a certain area on which the print image is to be printed. Therefore, if the exact location of the mobile image-forming device 100 cannot be identified, it is impossible to know which ink should be discharged to where the head 131 is currently located. Furthermore, the mobile image-forming device 100 needs to make a two-dimensional motion to print a two-dimensional image. Therefore, in order to identify the exact location of the mobile image-forming device 100, at least one location sensor 111 is needed for each of vertical and horizontal motions. That is, at least two location sensors 111 are needed in order to identify the location of the mobile image-forming device 100, and as illustrated in **FIG. 5****,** when the multiple location sensors 111-1, 111-2, 111-3, 111-4, 111-5, 111-6, and 111-7 are arranged, the location can be identified more exactly.

The method of identifying the location of the mobile image-forming device 100 is as follows. First, the skin surface initially touched by the mobile image-forming device 100 is set to coordinates (0,0). Then, the up/down motion and left/right motion of the mobile image-forming device 100 may be detected through each of the location sensors 111. As such, it is possible to know the initial coordinates and the amount of motion. Therefore, it is possible to identify the exact location of the mobile image-forming device 100.

In another example, it is possible to identify absolute location coordinates of the mobile image-forming device 100 by emitting laser light of a grid pattern on the skin surface on which a print image is to be printed.

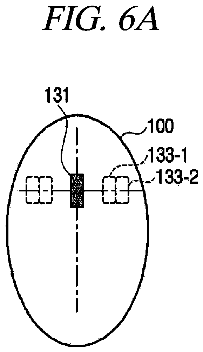

The coating unit 133 may spray a coating agent before and after the ink is discharged from the head 131. The coating unit 133 may be implemented as, for example, a roller, a dispenser, and the like. The reason for spraying a coating agent is that an image printed on skin or fabric, and the like may be degraded in print quality in terms of ink adhesion, color reproducibility, quick dryness, and wear resistance, compared to printing an image on paper. **FIG. 6a** through **FIG. 6C** illustrate various examples of the arrangement of the coating unit 133.

Referring to **FIG. 6A****,** a pair of PRE/POST coating units 133-1, 133-2 are arranged on each of the left and right of the head 131. Since the progress direction of the head 131 is not consistent, the coating unit 133 may be arranged on both the left and right of the head 131. For example, if the head 131 progresses to the right, the PRE coating unit 133-1 on the right sprays the coating agent before the head 131 arrives at the current location, and when the head 131 arrives at where the ink is discharged, the POST coating unit 133-2 on the left may spray the coating agent again.

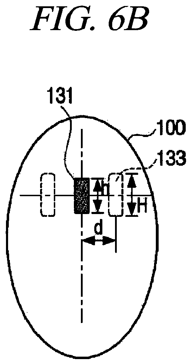

**FIG. 6B** is provided to explain the lengths of the head 131 and the coating unit 133 and the distance between the head 131 and the coating unit 133. Referring to **FIG. 6B****,** the length of the head 131 is denoted as h, the length of the coating unit 133 as H, and the distance between the head 131 and coating unit 133 as d.

The length of the coating unit 133 may be longer than that of the head 131. That is, a condition of H>h may be satisfied. When the length of the coating unit 133 is longer than that of the head 131, the area that the coating agent covers will be larger. Thus, the head 131 can discharge the ink above the area where the coating agent is deposited.

The distance d between the head 131 and coating unit 133 may be determined based on the drying time of the coating agent and the maximum printable speed. In order to suppress the mixing of the ink and the coating agent, the ink needs to be discharged after the coating agent is dried. Therefore, the head 131 and the coating unit 133 need to be separated from each other at least by a distance corresponding to the product of the maximum printable speed and the drying time of the coating agent.

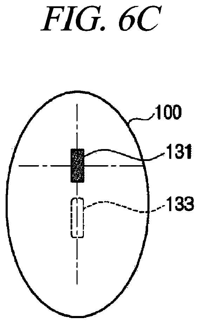

In another example, as shown in **FIG. 6C****,** the coating unit 133 may be arranged below the head 131. In the arrangement as shown in **FIG. 6C****,** it is possible to secure sufficient time for the coating agent to be dried. When the mobile image-forming device 100 moves over the skin area on which an image is to be printed for the first time, the coating unit 133 sprays the coating agent, but the head 131 does not discharge the ink. Thereafter, the head 131 starts discharging the ink when the head 131 arrives at where the coating agent is deposited, and, thus, a print image can be printed. For example, using the reflecting characteristics of the coating unit different from the reflecting characteristics of the skin, the mobile image-forming device 100 may determine whether it is an area where the coating agent is deposited, through the location sensor, and the like.

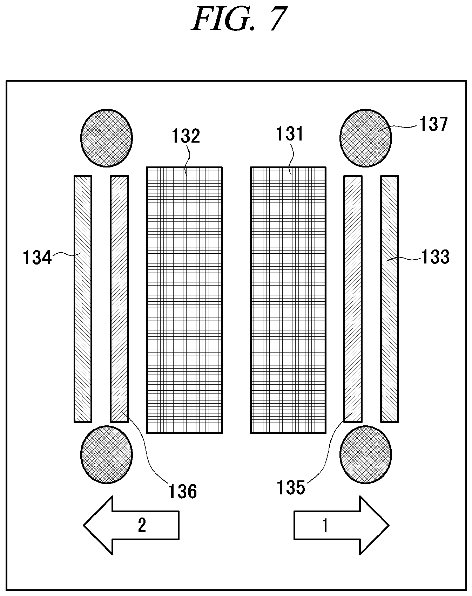

**FIG. 7** is provided to explain an air blower and a roller sensor according to an embodiment of the present disclosure.

As shown in the drawing, air blowers 135 and 136 may be further arranged between heads 131 and 132 and coating units 133 and 134, respectively. After the coating units 133 and 134 spray the coating agent or the heads 131 and 132 discharge the ink, the air blowers 135 and 136 blow air to the coating agent or the ink to more quickly dry the coating agent or the ink. In this case, the air blowers 135 and 136 are configured to discharge heat generated from various electronic circuit devices built in the mobile image-forming device 100 and thus function to discharge heat and further increase the drying speed of the coating agent. The air blowers 135 and 136 may be implemented including openings for discharging internal heat and air blower fans which are controlled to blow air at a predetermined time.

Specifically, if the mobile image-forming device 100 is moved to the right on the drawing, a first coating unit 133 sprays the coating agent and then, a first air blower 135 dries the coating agent. Thereafter, a first head 131 discharges the ink and then, a second head 132 may further discharge the ink. Then, a second air blower 136 dries the ink. Then, a second coating unit 134 sprays the coating agent to complete a print operation. On the contrary, if the mobile image-forming device 100 is moved to the left on the drawing, the second coating unit 134 sprays the coating agent and then, the second air blower 136 dries the coating agent. Thereafter, the second head 132 discharges the ink and then, the first head 131 may further discharge the ink. Then, the first air blower 135 dries the ink. Then, the first coating unit 133 sprays the coating agent to complete a print operation.

Meanwhile, **FIG. 7** illustrates an example where the two heads 131 and 132 are placed and the air blowers 135 and 136 and the coating units 133 and 134 are arranged on one sides of the heads 131 and 132, respectively, for convenience of description. The present disclosure is not necessarily limited thereto.

Meanwhile, a roller sensor 137 configured to detect a moving position of the mobile image-forming device 100, improve the mobility, and maintain a constant distance between skin and the mobile image-forming device 100 may be added near the head 131.

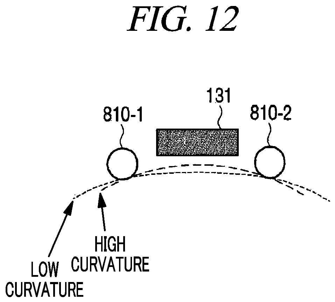

The roller sensor 137 may be configured in the form of a ball or wheel to improve the mobility of the mobile image-forming device 100. The roller sensor 137 may be configured including a ball and a sensor for tracking the movement of the ball like a ball mouse known as conventional technology. The roller sensor 137 may detect a change in position as the ball moves and track the position of the ball. Otherwise, the roller sensor 137 may be implemented using a wheel and an encoder for sensing the rotary frequency of the wheel. Thus, it is possible to easily check the moving direction or moving distance of the mobile image-forming device 100. Further, the mobile image-forming device 100 can be smoothly moved on the skin surface by lowering the friction coefficient on the skin surface. Further, due to the height of the roller sensor 137, a constant distance between the skin surface and the mobile image-forming device 100 can be maintained. As shown in the drawing, multiple roller sensors 137 may be arranged near the heads 131 and 132. The roller sensor 137 may function both as the above-described location sensor 111 and as a member 810 to be described below with reference to **FIG. 12****.**

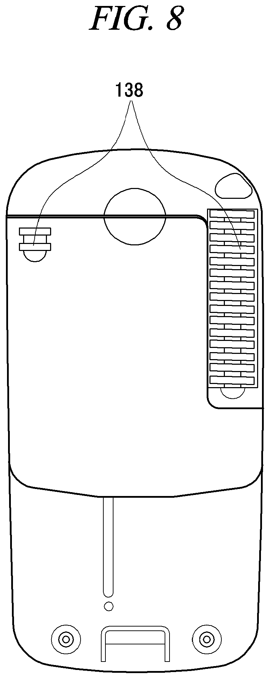

**FIG. 8** is provided to explain a roller sensor according to an embodiment of the present disclosure.

Unlike the ball-type roller sensor 137 shown in **FIG. 7****,** a wheel-type roller sensor 138 is arranged. That is, one or more roller sensors 138 may be arranged on one side of the mobile image-forming device 100 to be driven linearly. The roller sensor 138 may detect the rotary frequency or rotation direction of a wheel through a sensor such as an encoder to detect a movement state of the mobile image-forming device 100.

The display unit 140 may display image contents and a UI element that includes graphic objects. More specifically, the display unit 140 may display a print image, an image of a skin area on which a print image is to be printed, a data analyzing Ul, and an image searched online, and the like. The display unit 140 may be designed into various types of display panels. That is, the display unit 140 may be implemented by various display technologies, such as liquid crystal display (LCD), organic light-emitting diode (OLED), E-paper, plasma display panel (PDP), vacuum fluorescent display (VFD), field emission display (FED), electro luminescence display (ELD), and the like. The display panel is generally configured as a light-emitting type display panel, but does not exclude a reflective display panel. Furthermore, the display unit 140 may be configured as a flexible display, transparent display, and the like.

For example, the display unit 140 may be arranged on a top portion of the mobile image-forming device 100 and may display an image of a skin area on which a print image is to be printed. Further, the display unit 140 may combine a print image with a skin area and display the combined image in augmented reality. That is, the image processing unit 120 may combine the print image with the image of the skin area on which the print image is to be printed. Furthermore, the display unit 140 may display the combined image. Thus, the user may check the print result and adjust the size or location of the print image.

The display unit 140 may be implemented as a touch screen having a mutual layered structure with a touch pad, and the touch screen may be configured to detect the location and the area of a touch input and even the pressure of the touch input. In this case, the display unit 140 may perform the function of the user interface 150 that will be described below.

The user interface 150 enables the mobile image-forming device 100 to transmit and receive a command to and from the user. As described above, the user interface 150 may be implemented as a touch screen, but is not limited thereto. Instead, the user interface 150 may be implemented by voice recognition or motion recognition.

The user interface 150 may receive a user input for adjusting the location or size of an image displayed on the display unit 140. For example, the user may adjust the size of an image through a pinch-zoom operation. The image processing unit 120 may correct a print image in response to the received user input.

The camera 160 photographs an image. The mobile image-forming device 100 may use the image photographed by the camera 160 as a print image. In another example, the camera 160 may be implemented to substitute for the image sensor 113.

The communication interface 170 is configured as a wireless module so as to perform wireless communication with an external device. The communication interface 170 may include various communication chips, such as a Wi-Fi chip, Bluetooth chip, near field communication (NFC) chip, and a wireless communication chip. A Wi-Fi chip or Bluetooth chip may be used to transceive various connection information regarding service set identifier (SSID) or session keys and use the connection information for communication access and then transceive various information. The wireless communication chip refers to a chip for performing communication according to various communication standards, such as institute of electrical and electronics engineers (IEEE), ZigBee, 3rd generation (3G), 3rd generation partnership project (3GPP), and long term evolution (LTE).

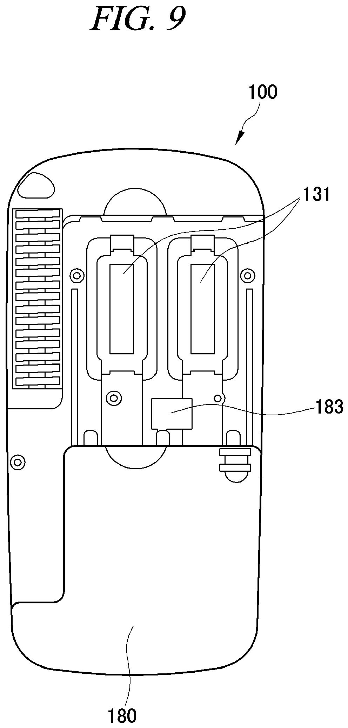

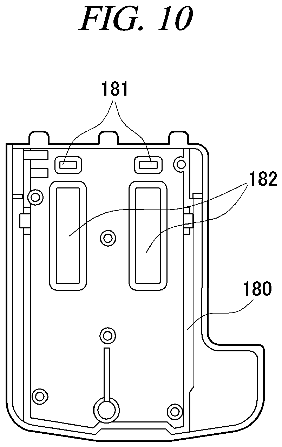

**FIG. 9** and **FIG. 10** are provided to explain a cleaning unit 180 according to an embodiment of the present disclosure.

In order to maintain the print quality, the head 131 needs to be maintained in an optimum state. In other words, ink remining on the head 131 may obstruct the discharge of ink and thus cause the generation of droplets or the clogging of the head 131.

To solve this problem, the mobile image-forming device 100 may further include the cleaning unit 180 that slidably moves between a capping position for capping the head 131 and an uncapping position for print. For example, the cleaning unit 180 may be slidably moved manually by the user, but is not limited thereto. The cleaning unit 180 may be slidably moved by a separate driver.

The cleaning unit 180 may include wiping units 181 that wipe ink from the head 131 while slidably moving in contact with the head 131. In other words, the cleaning unit 180 covers the head 131 to suppress the exposure of the head 131 to the outside during a period of non-use and removes ink remaining on the head 131 through the wiping units 181 during a sliding movement. For example, the wiping units 181 may be provided corresponding in number to the heads 131, but is not limited thereto.

Specifically, when the mobile image-forming device 100 is used, the cleaning unit 180 can be slidably moved from the capping position to the uncapping position. In this case, the wiping units 181 may wipe ink which has been leaked from the head 131 during a period of non-use. Further, when the use of the mobile image-forming device 100 is finished, the cleaning unit 180 can be slidably moved from the uncapping position to the capping position. The wiping units 181 may wipe ink remaining on the head 131. For example, the wiping units 181 may be formed of a porous absorber, such as fabric or sponge, capable of absorbing ink, but is not limited thereto.

The cleaning unit 180 may include capping units 182 which is brought into close contact with the head 131 when capping the head 131. For example, as shown in **FIG. 9****,** the capping units 182 are brought into close contact with the circumference of the head 131 and have a depressed central portion to collect ink dropped from the head 131 and thus suppress contamination with ink.

Further, the wiping units 181 and the capping units 182 may be replaced after use for a certain period of time if needed. For example, a replacement cycle may be indicated by means of an alarm through an application or its own alarm.

The cleaning unit 180 may further include an opening/closing sensor 183 that detects whether the cleaning unit 180 is opened or closed. For example, if the opening/closing sensor 183 detects that the cleaning unit is in an unpacked state where the head 131 is open to the outside for a certain period of time, an alarm may be given to the user to suppress drying of the head 131.

The mobile image-forming device 100 may perform a spitting operation for suppressing drying of the head 131 regularly or at the discretion of the user. The spitting operation can be performed after the opening/closing sensor 183 confirms that the cleaning unit 180 is at the capping position. For example, if the mobile image-forming device 100 is left without use for a long time, even when power is off, a predetermined amount of ink is discharged regularly through the head 131 to suppress a nozzle of the head 131. In this case, the discharged ink may be collected in the capping units 182.

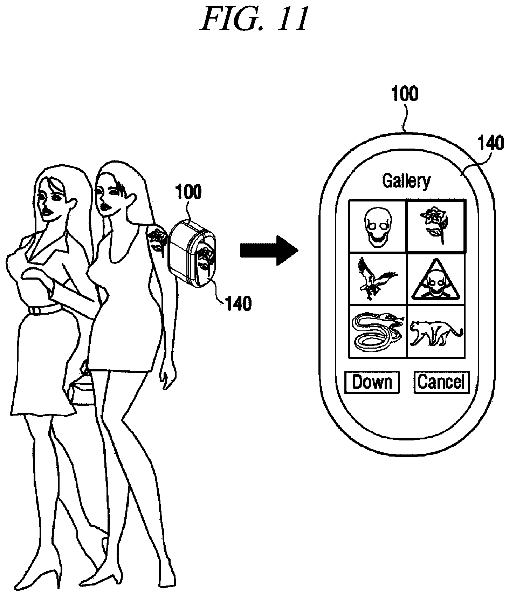

**FIG. 11** is provided to explain an example where an image similar to an image photographed by the camera 160 is searched online and used as a print image.

Referring to **FIG. 11****,** it is possible to photograph a tattoo on another person's body using the camera 160. The image being photographed in the camera 160 may be displayed on the display unit 140 in live view. The image processing unit 120 may control the communication interface 170 to remove the skin color from the photographed image and search online for an image that is similar to the image without the skin color. As shown in **FIG. 11****,** searched results of images are displayed on the display unit 140, and in response to a user command for selecting one of them, the selected image may be used as a print image.

The image processing unit 120 may analyze a phase difference of a color of the photographed image and automatically delete a skin color portion. Further, a user input for cropping only an image portion to be printed among the photographed image may be received through the user interface 150.

**FIG. 12** is provided to explain a member configured to maintain a constant distance between skin and the image forming unit 130 according to an embodiment of the present disclosure.

Since the mobile image-forming device 100 according to an embodiment of the present disclosure is based on an assumption that the mobile image-forming device 100 prints an image on a curved skin surface, the print environment is different from that of a general image-forming device where a constant distance is maintained between paper and an ink cartridge head.

If the distance between the skin on which a print image is to be printed and the head 131 is narrow, the skin may get burned by contact. On the contrary, if the distance between the skin and the head 131 is wide, the print quality may be degraded due to ink dispersion and an increase of dot size. Therefore, a certain distance between the skin and head 131 needs to be maintained even though the skin surface is curved.

**FIG. 12** illustrates members 810-1 and 810-2 for maintaining a constant distance between the head 131 and the skin. The members 810-1 and 810-2 may move the head 131 upwards according to high curvatures and move the head 131 downwards according to low curvatures. Accordingly, the mobile image-forming device 100 may maintain a constant distance between the head 131 and the skin even if there is a curvature on a print area.

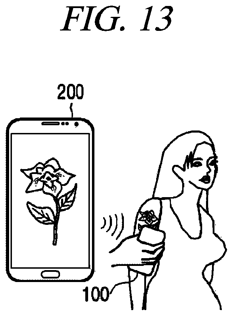

**FIG. 13** is a conceptual diagram provided to explain the mobile image-forming device 100 which operates and interworks with a mobile device 200 according to another embodiment of the present disclosure.

In another embodiment of the present disclosure, a print image may be selected in the mobile device 200. Further, the mobile device 200 may transmit the selected image to the mobile image-forming device 100. The mobile image-forming device 100 may print the received print image after correcting the received print image to be suitable for the surface condition.

In yet another embodiment of the present disclosure, the mobile device 200 may further perform the function of correcting the print image. The mobile device 200 may transmit the corrected print image to the mobile image-forming device 100, and the mobile image-forming device 100 may perform only the print function.

According to the above-described various embodiments of the present disclosure, it is possible to correct image data according to the condition of the skin on which a print image is to be printed, so that the image being printed is not different in color and shape from the original print image.

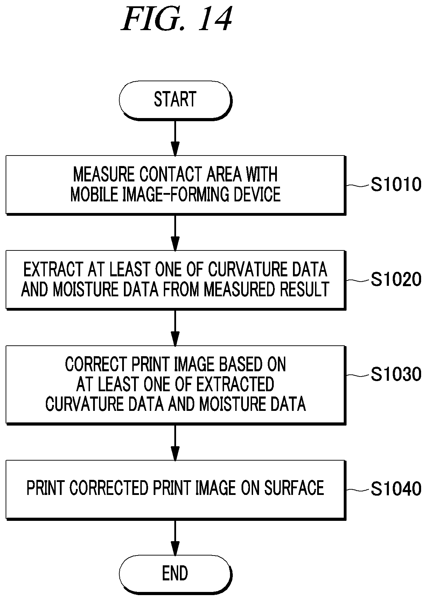

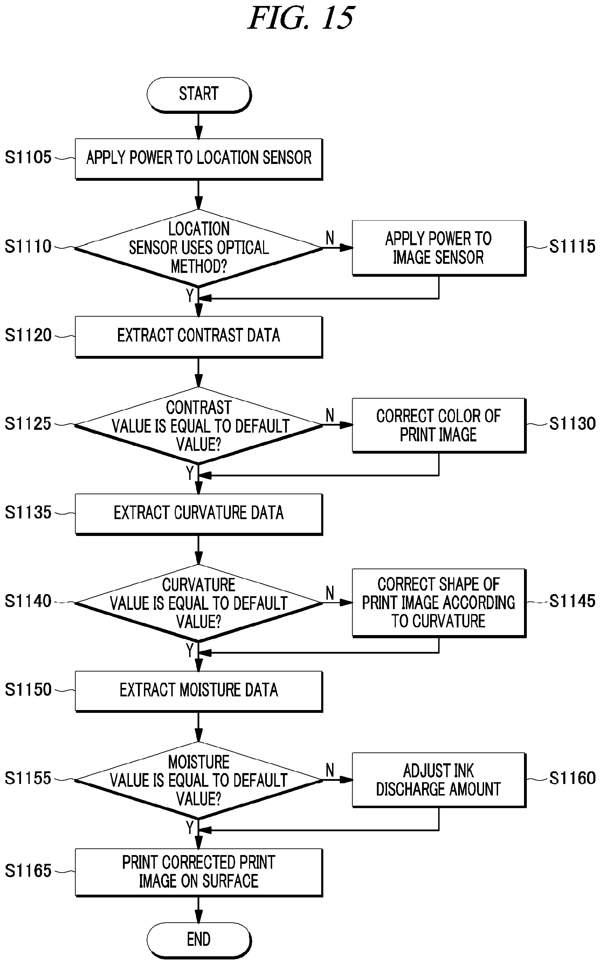

**FIG. 14** and **FIG. 15** are flowcharts provided to explain an image correcting method of the mobile image-forming device 100 according to various embodiments of the present disclosure.

Referring to **FIG. 14****,** the mobile image-forming device 100 may measure a contact area with the mobile image-forming device 100 in a surface area on which a print image is to be printed (S1010). The mobile image-forming device 100 may measure the contrast, color tone, and curvature of the contact area. Further, the mobile image-forming device 100 may measure a moisture content or hydrogen ion concentration of the contact area.

Furthermore, the mobile image-forming device 100 may extract at least one of curvature data and moisture data from the measured result (S1020). In addition to the curvature data and moisture data, the mobile image-forming device 100 may further extract contrast data. The mobile image-forming device 100 may photograph an image of the contact area, analyze the photographed image, and extract at least one of the contrast data and curvature data.

Thereafter, the mobile image-forming device 100 may correct the print image based on at least one of the extracted curvature data and moisture data (S1030). For example, the mobile image-forming device 100 may correct the color of the print image using a pre-stored conversion table based on the extracted contrast data. If the brightness of the extracted contrast data is low, the mobile image-forming device 100 may correct the brightness of the print image by increasing the discharge amount of W ink among C, M, Y, W inks. In another example, the mobile image-forming device 100 may detect the curvature of the print area and perform a three-dimensional rendering on a two-dimensional print image according to the detected curvature. In yet another example, the mobile image-forming device 100 may correct an ink discharge amount for the print image based on the measured moisture content or hydrogen ion concentration. The mobile image-forming device 100 may correct the ink discharge amount to increase as the moisture content decreases and the hydrogen ion concentration increases.

Lastly, the mobile image-forming device 100 may print the corrected print image on the surface (S1040).

**FIG. 15** is a flowchart illustrating an embodiment in consideration of power and the sequence of operations of each sensor included in the mobile image-forming device 100 according to another embodiment of the present disclosure.

First, after contacting a location on which a print image is to be printed, the mobile image-forming device 100 applies power to a location sensor (S1105). The location sensor may determine relative motions of the mobile image-forming device 100 into two-dimensional coordinates based on the location initially touched by the mobile image-forming device 100. If the location sensor is capable of photographing the condition of the skin on which a print image is to be printed by using an optical method (S1110-Y), an image sensor may not be used. Therefore, in the case where the location sensor does not use the optical method (1110-N), the mobile image-forming device 100 applies power to the image sensor (S1115).

Thereafter, the mobile image-forming device 100 extracts the skin contrast data of the area on which a print image is to be printed using the image sensor or location sensor (S1120). The mobile image-forming device 100 compares the contrast value with a default value to determine whether the color of the print image needs to be corrected (S1125). For example, in the case where the color of the print image needs to be corrected (S1125-N), the mobile image-forming device 100 may correct the color of the print image using the pre-stored conversion table (S1130).

Thereafter, the mobile image-forming device 100 extracts curvature data (S1135). Likewise, the mobile image-forming device 100 compares the curvature value with a default value to determine whether the shape needs to be corrected (S1140). For example, in the case where there is no curvature or the curvature value is close to that of a plane surface (S1140-Y), the mobile image-forming device 100 may not perform correction according to the curvature. If not (S1140-N), the mobile image apparatus 100 corrects the shape of the print image according to the curvature (S1145).

Thereafter, the mobile image-forming device 100 extracts moisture data (S1150). The mobile image-forming device 100 compares the moisture value with a default value to determine whether the ink discharge amount for the print image needs to be adjusted (S1155). For example, the mobile image-forming device 100 may set normal skin as the default value. In this case, if the skin type is determined as oily or dry (S1155-N), the mobile image-forming device adjusts the ink discharge amount (S1160). The mobile image-forming device 100 may decrease the ink discharge amount in case of oily skin and increase the ink discharge amount in case of dry skin.

Lastly, the mobile image-forming device 100 prints the corrected print image on the surface (S1165). However, the sequence of corrections to the print image using contrast data, curvature data, and moisture data is not necessarily limited to the illustration of **FIG. 15****.**

Besides, various embodiments of the image correcting method of the mobile image-forming device 100 overlap with the explanation made in the embodiments of the mobile image-forming device 100, and thus redundant explanation will not be provided.

The above-described methods may be implemented in an executable program command form by various computer means and be recorded in a computer-readable storage medium. The computer-readable storage medium may include a program command, a data file, and a data structure individually or a combination thereof. The program command recorded in the medium may be specially designed or configured for the present disclosure or may be known to those skilled in a computer software field to be used. Examples of the computer-readable storage medium include magnetic media such as hard disk, floppy disk, or magnetic tape, optical media such as CD-ROM or DVD, magneto-optical media such as floptical disk, and a hardware device such as ROM, RAM, flash memory specially configured to store and execute program commands. Examples of the program command include a machine language code created by a complier and a high-level language code executable by a computer using an interpreter. The hardware device may be configured to be operated as at least one software module to perform an operation of the present disclosure, and vice versa.

Although the present disclosure has been described by limited exemplary embodiments and drawings, the present disclosure is not limited to the exemplary embodiment. Various modifications and changes can be made by a person with ordinary skill in the art from the descriptions above.

Therefore, the scope of the present disclosure is not limited to the embodiments described above but should be defined by the following claims.

### [EXPLANATION OF REFERENCE NUMERALS]

- 100:: Mobile image-forming device
- 110:: Surface measuring unit
- 120:: Image processing unit
- 130:: Image forming unit
- 140:: Display unit
- 150:: User interface
- 160:: Camera
- 170:: Communication interface

## Claims

1. A mobile image-forming device (100), comprising:
a surface measuring unit (110) configured to measure a contact area with the mobile image-forming device (100) in a surface area on which a print image is to be printed;
**characterized by** an image processing unit (120) configured to extract moisture data from the measured result and correct an ink discharge amount for the print image based on the moisture data; and
an image forming unit (130) configured to print the print image on the surface depending on the corrected ink discharge amount.

2. The mobile image-forming device (100) of Claim 1,
wherein the surface measuring unit (110) includes:
a moisture sensor (115) configured to measure a moisture content or hydrogen ion concentration (pH) of the contact area, and
the image processing unit (120) corrects an ink discharge amount for the print image based on the measured moisture content or hydrogen ion concentration.

3. The mobile image-forming device (100) of Claim 1,
wherein the surface measuring unit (110) includes:
a location sensor (111) configured to determine the contact area with the mobile image-forming device (100),
wherein the location sensor (111) is arranged on a horizontal and vertical line of where the image forming unit (130) is placed, and
a two-dimensional motion of the mobile image forming device (100) is detected using the arranged location sensor (111).

4. The mobile image-forming device (100) of Claim 1,
wherein the image forming unit (130) further includes:
an air blower (135) configured to dry the ink or the coating agent.

5. The mobile image-forming device (100) of Claim 1, further comprising:
a cleaning unit (180) configured to slidably move between a capping position for capping a head (131) included in the image forming unit (130) and an uncapping position for print,
wherein the cleaning unit (180) includes:
wiping units (181) configured to wipe ink from the head (131) while slidably moving in contact with the head (131); and
capping units (182) configured to be brought into close contact with the head (131) when capping the head (131).

6. The mobile image-forming device (100) of Claim 1, further comprising:
a display unit (140); and
a user interface (150) configured to receive a user input,
wherein the image processing unit (120) controls the display unit (140) to compose the print image on the surface area on which the print image is to be printed and to display the composed image, and
when a user input for adjusting a location or size of the displayed image is received, the image processing unit (120) corrects the print image in response to the received user input.

7. The mobile image-forming device (100) of Claim 1, further comprising:
a camera (160) configured to photograph an image, and
a communication interface (170),
wherein the image processing unit (120) uses the photographed image as the print image or controls the communication interface (170) to remove a skin color from the photographed image and to search online for an image that is similar to the image from which the skin color has been removed, and the searched image is used as the print image.

8. An image correcting method of a mobile image-forming device (100), comprising:
measuring a contact area with the mobile image-forming device (100) in a surface area on which a print image is to be printed;
**characterized by** extracting moisture data from the measured result;
correcting an ink discharge amount for the print image based on the moisture data; and
printing the print image on the surface depending on the corrected ink discharge amount;

9. The image correcting method of Claim 8,
wherein the measuring further includes:
measuring a moisture content or hydrogen ion concentration (pH) of the contact area, and
the correcting includes correcting an ink discharge amount for the print image based on the measured moisture content or hydrogen ion concentration.

## Patentansprüche

1. Mobile Bilderzeugungsvorrichtung (100), welche umfasst:
eine Oberflächenmesseinheit (110), die dazu ausgebildet ist, eine Kontaktfläche mit der mobilen Bilderzeugungsvorrichtung (100) in einem Oberflächenbereich zu messen, auf den ein Druckbild gedruckt werden soll; **gekennzeichnet durch** eine Bildbearbeitungseinheit (120), die dazu ausgebildet ist, dem gemessenen Ergebnis Feuchtigkeitsdaten zu entnehmen und eine Tintenabgabemenge für das Druckbild auf der Grundlage der Feuchtigkeitsdaten zu korrigieren; und
eine Bilderzeugungseinheit (130), die dazu ausgebildet ist, abhängig von der korrigierten Tintenabgabemenge das Druckbild auf die Oberfläche zu drucken.

2. Mobile Bilderzeugungsvorrichtung (100) nach Anspruch 1,
wobei die Oberflächenmesseinheit (110) enthält:
einen Feuchtigkeitssensor (115), der dazu ausgebildet ist, einen Feuchtigkeitsgehalt oder eine Wasserstoffionenkonzentration (pH) der Kontaktfläche zu messen, und
die Bildbearbeitungseinheit (120) eine Tintenabgabemenge für das Druckbild auf der Grundlage des/der gemessenen Feuchtigkeitsgehalts oder Wasserstoffionenkonzentration korrigiert.

3. Mobile Bilderzeugungsvorrichtung (100) nach Anspruch 1,
wobei die Oberflächenmesseinheit (110) enthält:
einen Ortssensor (111), der dazu ausgebildet ist, die Kontaktfläche mit der mobilen Bilderzeugungsvorrichtung (100) zu bestimmen,
wobei der Ortssensor (111) auf einer horizontalen und vertikalen Linie angeordnet ist, von wo die Bilderzeugungseinheit (130) platziert ist, und
eine zweidimensionale Bewegung der mobilen Bilderzeugungsvorrichtung (100) unter Verwendung des angeordneten Ortssensors (111) ermittelt wird.

4. Mobile Bilderzeugungsvorrichtung (100) nach Anspruch 1,
wobei die Bilderzeugungseinheit (130) zudem enthält:
ein Luftgebläse (135), das dazu ausgebildet ist, die Tinte oder das Beschichtungsmittel zu trocknen.

5. Mobile Bilderzeugungsvorrichtung (100) nach Anspruch 1, welche zudem umfasst:
eine Reinigungseinheit (180), die dazu ausgebildet ist, sich gleitend zwischen einer Abdeckungsposition zum Abdecken eines in der Bilderzeugungseinheit enthaltenen Kopfes (131) und einer Aufdeckungsposition zum Drucken zu bewegen,
wobei die Reinigungseinheit (180) enthält:
Wischeinheiten (181), die dazu ausgebildet sind, Tinte von dem Kopf (131) abzuwischen, während sie sich gleitend in Kontakt mit dem Kopf (131) bewegen; und
Abdeckeinheiten (182), die dazu ausgebildet sind, in engen Kontakt mit dem Kopf (131) gebracht zu werden, wenn sie den Kopf (131) abdecken.

6. Mobile Bilderzeugungsvorrichtung (100) nach Anspruch 1, welche zudem umfasst:
eine Anzeigeeinheit (140); und
eine Benutzerschnittstelle (150), die dazu ausgebildet ist, eine Benutzereingabe zu empfangen,
wobei die Bildbearbeitungseinheit (120) die Anzeigeeinheit (140) steuert,
um das Druckbild auf dem Oberflächenbereich, auf den das Bild gedruckt werden soll, zusammenzusetzen und das zusammengesetzte Bild anzuzeigen, und,
wenn eine Benutzereingabe zum Einstellen eines Ortes oder einer Größe des angezeigten Bildes empfangen wird, die Bildbearbeitungseinheit (120) das Druckbild als Reaktion auf die empfangene Benutzereingabe korrigiert.

7. Mobile Bilderzeugungsvorrichtung (100) nach Anspruch 1, welche zudem umfasst:
eine Kamera (160), die dazu ausgebildet ist, ein Bild zu fotografieren, und
eine Kommunikationsschnittstelle (170),
wobei die Bildbearbeitungseinheit (120) das fotografierte Bild als das Druckbild verwendet oder die Kommunikationsschnittstelle (170) steuert,
um eine Hautfarbe aus dem fotografierten Bild zu entfernen und online nach einem Bild zu suchen, das ähnlich zu dem Bild ist, aus dem die Hautfarbe entfernt worden ist, und das gesuchte Bild als das Druckbild verwendet wird.

8. Bildkorrekturverfahren einer mobilen Bilderzeugungsvorrichtung (100), welches umfasst:
Messen einer Kontaktfläche mit der mobilen Bilderzeugungsvorrichtung (100) in einem Oberflächenbereich, auf den ein Druckbild gedruckt werden soll;
**gekennzeichnet durch** Entnehmen von Feuchtigkeitsdaten aus dem gemessenen Ergebnis;
Korrigieren einer Tintenabgabemenge für das Druckbild auf der Grundlage der Feuchtigkeitsdaten; und,
Drucken des Druckbildes auf die Oberfläche, abhängig von der korrigierten Tintenabgabemenge.

9. Bildkorrekturverfahren nach Anspruch 8,
wobei das Messen zudem enthält:
Messen eines Feuchtigkeitsgehalts oder einer
Wasserstoffionenkonzentration (pH) der Kontaktfläche, und
das Korrigieren ein Korrigieren einer Tintenabgabemenge für das Druckbild auf der Grundlage des/der gemessenen Feuchtigkeitsgehalts oder Wasserstoffionenkonzentration enthält.

## Revendications

1. Dispositif mobile de formation d'image (100), comprenant :
une unité de mesure de surface (110) configurée pour mesurer une zone de contact avec le dispositif mobile de formation d'image (100) dans une zone de surface sur laquelle une image à imprimer doit être imprimée ;
**caractérisé par** une unité de traitement d'image (120) configurée pour extraire des données d'humidité du résultat mesuré et corriger une quantité de décharge d'encre pour l'image à imprimer sur la base des données d'humidité ; et
une unité de formation d'image (130) configurée pour imprimer l'image à imprimer sur la surface en fonction de la quantité de décharge d'encre corrigée.

2. Dispositif mobile de formation d'image (100) selon la revendication 1,
dans lequel l'unité de mesure de surface (110) inclut :
un capteur d'humidité (115) configuré pour mesurer une teneur en humidité ou une concentration en ions hydrogène (pH) de la zone de contact, et
l'unité de traitement d'image (120) corrige une quantité de décharge d'encre pour l'image à imprimer en fonction de la teneur en humidité ou de la concentration en ions hydrogène mesurée.

3. Dispositif mobile de formation d'image (100) selon la revendication 1,
dans lequel l'unité de mesure de surface (110) inclut :
un capteur de localisation (111) configuré pour déterminer la zone de contact avec le dispositif mobile de formation d'image (100),
dans lequel le capteur de localisation (111) est agencé sur une ligne horizontale et verticale de l'endroit où l'unité de formation d'image (130) est placée, et
un mouvement bidimensionnel du dispositif mobile de formation d'image (100) est détecté en utilisant le capteur de localisation agencé (111).

4. Dispositif mobile de formation d'image (100) selon la revendication 1,
dans lequel l'unité de formation d'image (130) inclut en outre :
une soufflerie d'air (135) configurée pour sécher l'encre ou l'agent de revêtement.

5. Dispositif mobile de formation d'image (100) selon la revendication 1, comprenant en outre :
une unité de nettoyage (180) configurée pour se déplacer par glissement entre une position de coiffement pour coiffer une tête (131) incluse dans l'unité de formation d'image (130) et une position de décoiffement pour l'impression,
dans lequel l'unité de nettoyage (180) inclut :
des unités d'essuyage (181) configurées pour essuyer l'encre de la tête (131) tout en se déplaçant par glissement en contact avec la tête (131) ; et
des unités de coiffement (182) configurées pour être amenées en contact étroit avec la tête (131) lors du coiffement de la tête (131).

6. Dispositif mobile de formation d'image (100) selon la revendication 1, comprenant en outre :
une unité d'affichage (140) ; et
une interface utilisateur (150) configurée pour recevoir une entrée utilisateur,
dans lequel l'unité de traitement d'image (120) commande l'unité d'affichage (140) pour composer l'image à imprimer sur la zone de surface sur laquelle l'image à imprimer doit être imprimée et pour afficher l'image composée, et lorsqu'une entrée utilisateur pour ajuster une localisation ou une taille de l'image affichée est reçue, l'unité de traitement d'image (120) corrige l'image à imprimer en réponse à l'entrée utilisateur reçue.

7. Dispositif mobile de formation d'image (100) selon la revendication 1, comprenant en outre :
un appareil photo (160) configuré pour photographier une image, et
une interface de communication (170),
dans lequel l'unité de traitement d'image (120) utilise l'image photographiée comme image à imprimer ou commande l'interface de communication (170) pour supprimer une couleur de peau de l'image photographiée et pour rechercher en ligne une image qui est similaire à l'image de laquelle la couleur de peau a été supprimée, et l'image recherchée est utilisée comme image à imprimer.

8. Procédé de correction d'image d'un dispositif mobile de formation d'image (100), comprenant :
la mesure d'une zone de contact avec le dispositif mobile de formation d'image (100) dans une zone de surface sur laquelle une image à imprimer doit être imprimée ;
**caractérisé par**
l'extraction de données d'humidité du résultat mesuré ;
la correction d'une quantité de décharge d'encre pour l'image à imprimer sur la base des données d'humidité ; et
l'impression de l'image à imprimer sur la surface en fonction de la quantité de décharge d'encre corrigée.

9. Procédé de correction d'image selon la revendication 8,
dans lequel la mesure inclut en outre :
la mesure d'une teneur en humidité ou d'une concentration en ions hydrogène (pH) de la zone de contact, et
la correction inclut la correction d'une quantité de décharge d'encre pour l'image à imprimer sur la base de la teneur en humidité ou de la concentration en ions hydrogène mesurée.
